(19) Europäisches Patentamt
European Patent Office
Office européen des brevets

(11) Veröffentlichungsnummer: **0 003 335**
**B1**

# (12) EUROPÄISCHE PATENTSCHRIFT

(45) Veröffentlichungstag der Patentschrift: **01.07.81**

(21) Anmeldenummer: **79100161.3**

(22) Anmeldetag: **19.01.79**

(51) Int. Cl.$^3$: **C 07 C 121/43**
//C07C131/00

## (54) Verfahren zur Herstellung von Aminomalonsäuredinitriltosylat oder Acetylaminomalonsäuredinitril.

(30) Priorität: **20.01.78 CH 611/78**

(43) Veröffentlichungstag der Anmeldung:
**08.08.79 Patentblatt 79/16**

(45) Bekanntmachung des Hinweises auf die Patenterteilung:
**01.07.81 Patentblatt 81/26**

(84) Benannte Vertragsstaaten:
**BE DE FR GB IT LU NL**

(56) Entgegenhaltungen:
**US - A - 3 670 007**

**HOUBEN-WEYL "Methoden der organischen Chemie"**
**4.Auflage, Band XI, Teil 1, 1957**
**GEORG THIEME VERLAG, Stuttgart Seiten 498, 531 bis 532**

(73) Patentinhaber: **LONZA AG**
**Gampel/Wallis (CH)**

(72) Erfinder: **Junek, Hans, Prof.Dr.**
**Herrandgasse 22**
**Graz (AT)**
Erfinder: **Mittelbach, Martin, Dr.**
**Heinrichstrasse 33**
**Graz (AT)**

(74) Vertreter: **von Füner, Alexander, Dr. et al,**
**Patentanwälte Schiff, von Füner Strehl, Schübel-Hopf, Ebbinghaus, Finck Mariahilfplatz 2 u. 3**
**D-8000 München 90 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Verfahren zur Herstellung von Aminomalonsäuredinitriltosylat oder Acetylaminomalonsäuredinitril

Die Erfindung betrifft ein Verfahren zur Herstellung von Aminomalonsäuredinitril derivaten.

Es ist bekannt, Aminomalodinitril als p-Toluolsulfat durch Reduktion von Oximinomalonsäuredinitril mit Aluminiumamalgan zu isolieren. Diese Herstellung ist jedoch langwierig und umständlich. Die Amalgamierung des Aluminiums bereitet Schwierigkeiten, da die Quecksilberschicht auf der Oberfläche des Aluminiums nicht gut haftet. Außerdem bleibt beim Absaugen des Aluminiumhydroxids nach der Reduktion viel Produkt im Niederschlag zurück (J. P. Ferris, L. E. Orgel, J.Am.Chem.Soc. *87*, 4976—7 (1965); J. P. Ferris, L. E. Orgel, J.Am.Chem.Soc. *88*, 3829—31 (1966); J. P. Ferris US-Patent 3 670 007 (1972), C.A. *77*, 100866v (1972); J. P. Ferris, R. A. Sanchez, R. W. Mancuso, Org. Synth. *48*, 1—3).

J. P. Ferris beschreibt in seinem Patent auch Reduktionen mit Zink und Natriumdithionit, wobei er sich jedoch auf den qualitativen Nachweis von Aminomalonsäuredinitril in der Reaktionslösung beschränkt.

Aufgabe der Erfindung ist ein Verfahren zur Herstellung von Aminomalonsäuredinitril als Derivate, das es erlaubt, die Nachteile des Verfahrens mit Aluminiumamalgam zu vermeiden und auf einfache Weise die Produkte herzustellen.

Erfindungsgemäß wird das durch ein Verfahren erreicht, wobei man von einer Malonsäuredinitrilverbindung der allgemeinen Formel

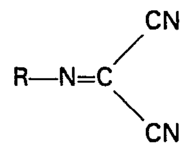

ausgeht, wobei R = —OH oder

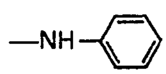

bedeutet, und welches dadurch gekennzeichnet ist, daß man in Gegenwart von Raneykatalysatoren als Reduktionsmittel bei Wasserstoffdrücken von ~ 5 bis 7 bar (4 bis 6 atü) und Temperaturen von 10 bis 80°C in Tetrahydrofuran als Lösungsmittel arbeitet und das Aminomalonsäuredinitril durch Behandlung mit p-Toluolsulfonsäure aus der Reaktionslösung ausfällt und als Tosylat isoliert oder in Acetanhydrid als Lösungsmittel zum Aminomalonsäuredinitril umsetzt und dieses als Acetylaminomalonsäuredinitril ausfällt und isoliert.

Geeignete Ausgangsverbindungen sind demnach Oximinomalonsäuredinitril und Phenylazomalonsäuredinitril.

Die Reaktion kann beispielsweise ausgeführt werden, indem man Oximinomalonsäuredinitril aus Malodinitril in Eisessig mit Natriumnitrit, gelöst in Wasser, bei Temperaturen um 0°C umsetzt und mit Äther extrahiert. Das Reaktionsgemisch kann dann mit Tetrahydrofuran versetzt und der Äther abdestilliert werden. Die Oximinomalonsäuredinitril enthaltende Tetrahydrofuranlösung wird erfindungsgemäß weiter umgesetzt.

Den Raneykatalysator, der zweckmäßig Ni, Fe, Cu und/oder Co enthält, gibt man zur Tetrahydrofuranlösung und reduziert bei den genannten Wasserstoffdrücken und Temperaturen in einer Zeit von 1 bis 10 Stunden.

In einer zweckmäßigen Ausführungsform wendet man 60 bis 80 g, vorzugsweise 70 g, Katalysator pro Mol Malonsäuredinitril an.

Nach Entfernen des Katalysators gibt man p-Toluolsulfonsäure in Äther zum Reaktionsgemisch. Man kann dabei ein-oder zweistufig arbeiten, wobei die letztere Arbeitsweise bevorzugt ist. Danach verwirft man den ersten Verunreinigungen enthaltenden Niederschlag. Das Filtrat versetzt man mit weiterer p-Toluolsulfonsäure in Äther und nach ca. 1 bis 20 Stunden läßt sich der entstandene Niederschlag von Aminomalondinitril-p-toluolsulfonat aus dem Reaktionsgemisch isolieren. In der vorzugsweisen Ausführung gibt man also die p-Toluolsulfonsäure in zwei Fraktionen zum Reaktionsgemisch zu, wobei in der ersten Fraktion 0,02 bis 0,03 Mol p-Toluolsulfonsäure und in der zweiten Fraktion 0,03 bis 0,1 Mol p-Toluolsulfonsäure, bezogen auf 0,1 Mol Malonsäuredinitril, angewendet werden können.

Wird Phenylazomalonsäuredinitril in Tetrahydrofuran mit Raneynickel reduziert, entstehen als Endprodukte Anilin und Aminomalonnitril, die gemeinsam als p-Toluolsulfonate gefällt werden können. Bei Verwendung von Acetanhydrid als Lösungsmittel entstehen die Acetylderivate der beiden Amine.

Beispiel 1

Herstellung der Oximinomalonsäuredinitrils:

6,6 g (0,1 Mol) Malodinitril werden in 20 ml Eisessig gelöst und mit einer Eiskochsalzmischung auf 0°C abgekühlt. Eine Lösung von 12,5 g (0,18 Mol) Natriumnitrit in 20 ml Wasser wird während 30 Minuten bei einer Temperatur von 0°C hinzugetropft. Man läßt 4 Stunden unter Eiskühlung weiterrühren und extrahiert anschließend 2 mal mit je 50 ml Äther. Die ätherische Lösung trocknet man mit Natriumsulfat, filtriert und versetzt mit 100 ml abs. Tetrahydrofuran. Danach wird durch Anlegen von Vakuum (12 mm Hg, kein Erhitzen notwendig) der Äther (100 ml) abdestilliert.

Herstellung des Aminomalonsäuredinitrils:

Die Tetrahydrofuran-Lösung wird anschließend mit 7 g Raneynickel (puriss., Fluka) bei einem Wasserstoffdruck von ~ 5 bar 4 Stunden bei 20°C reduziert. Nach Absaugen des Katalysators und Einengen auf 100 ml gibt man 5 g (26,3 mMol) p-Toluolsulfonsäure in 20 ml Äther hinzu und verwirft den entstandenen Niederschlag. Das Filtrat versetzt man mit weiteren 15 g p-Toluolsulfonsäure in 50 ml Äther, füllt mit Äther auf 250 ml auf und läßt 12 Stunden bei ca. 4°C stehen. Den entstandenen Niederschlag von Aminomalonnitril-p-toluolsulfonat saugt man ab und wäscht mit eiskeltem Acetonitril und Äther nach. Man trocknet im Vakuumexsiccator bei 20°C.

Ausbeute 12,2 g (49% d.Th.), Smp. 165°C.

Umkristallisation: Acetonitril Smp. 175°C (vgl. US—PS 3 670 007, Sp. 5,Z.41 und Sp.7,Z.4)

$C_{10}H_{11}N_3O_3S$ (253,3)

| | | | | |
|---|---|---|---|---|
| Ber. | C 47,42 | H, 4,38 | N 16,59 | S 12,66 |
| Gef. | C 47,32 | H, 4,44 | N 15,98 | S 13,33 |

$^1$H—NMR (DMSO): 2,23 ppm (s,$CH_3$), 6,00 ppm (s,CH),8,10 ppm (s,$NH^+{}_3$), 7,22 ppm (q, aromat.)

Beispiel 2

5,1 Phenylazomalodinitril (0,03 Mol) werden in 100 ml absolutem Tetrahydrofuran gelöst und mit ca. 10 g Raneynickel versetzt. Anschließend reduziert man bei einem Wasserstoffdruck von ~ 5 bar 6 Stunden bei 22°C. Nach Absaugen des Katalysators fügt man zum Filtrat 20 g (0,13 Mol) p-Toluolsulfonsäure in 50 ml Äther hinzu und saugt den entstandenen Niederschlag aus Aminomalonnitril- und Anilin-p-toluolsulfonat ab.

Ausbeute: 5 g (31% d.Th.).

Beispiel 3

5,1 g (0,03 Mol) Phenylazomalonnitril werden in 100 ml Acetanhydrid gelöst und mit ca. 10 g Raneynickel versetzt. Man reduziert bei einem Wasserstoffdruck von ~ 5 bar 6 Stunden bei 35°C. Der Katalysator wird abgesaugt, das Filtrat über eine Vorrichtung zur Vakuumdestillation geleitet, deren Blase sich um ihre eigene Achse dreht und sich das zu destillierende Medium in dünner Schicht auf der ganzen inneren Oberfläche der Blase verteilt (ROTAVAPOR Markenzeichen) und das entstehende Öl mit Wasser angerieben. Der sich bildende Niederschlag besteht aus einer Mischung von Acetylaminomalonitril und Acetanilid und kann im Dünnschichtchromatogramm (Eisessig:Benzol—1:10) auggetrennt werden.

Ausbeute: 2,5 g (27% d.Th.).

## Patentansprüche

1. Verfahren zur Herstellung von Aminomalonsäuredinitriltosylat oder Acetylaminomalonsäuredinitril aus einer Malonsäuredinitrilverbindung der allgemeinen Formel

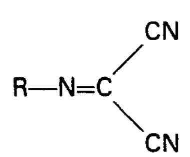

wobei R = —OH oder

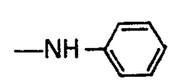

bedeutet, durch Reduktion, dadarch gekennzeichnet, daß man in Gegenwart von Raneykatalysatoren als Reduktionsmittel bei Wasserstoffdrücken von ~ 5 bis 7 bar (4 bis 6 atü) und Temperaturen von 10 bis 80°C in Tetrahydrofuran als Lösungsmittel arbeitet und das Aminomalonsäuredinitril durch Behandlung mit p-Toluolsulfonsäure aus der Reaktionslösung ausfällt und als Tosylat isoliert oder in Acetanhydrid

als Lösungsmittel zum Aminomalonsauredinitril umsetzt und dieses als Acetylaminomalonsäuredinitril ausfällt und isoliert.

2. Verfahren gemäß Patentanspruch 1, dadurch gekennzeichnet, daß man Raneykatalysatoren, die Ni, Fe, Cu und/oder Co enthalten, anwendet.

3. Verfahren gemäß Patentansprüchen 1 und 2, dadurch gekennzeichnet, daß man 60 bis 80 g, vorzugsweise 70 g, Katalysator pro Mol Malonsäuredinitrilverbindung anwendet.

4. Verfahren gemäß Patentansprüchen 1 bis 3, dadurch gekennzeichnet, daß man die p-Toluolsulfonsäure in zwei Fraktionen zum Reaktionsgemisch gibt, wobei man in der ersten Fraktion 0,02 Mol bis 0,03 Mol p-Toluolsulfonsäure und in der zweiten Fraktion 0,03 Mol bis 0,1 Mol p-Toluolsulfonsäure, bezogen auf 0,1 Mol Malonsäuredinitrilverbindung, anwendet.

**Revendications**

1. Procédé de préparation du tosylate d'aminomalonitrile ou de l'acétylaminomalonitrile par réduction á partir d'un composé du malonitrile de formule générale:

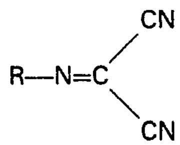

dans laquelle:

R représente —OH ou

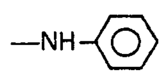

caractérisé en ce que l'on opére en présence de catalyseurs de Raney comme réducteurs á des pressions de vapeur d'environ 5 á 7 bars (4 á 6 atü) et á des températures de 10 á 80°C dans le tétrahydrofuranne comme solvant, que l'on provoque la précipitation de l'aminomalonitrile par traitement par l'acide p-toluéne-sulfonique et l'isole sous forme de tosylate ou qu'on le transforme, dans l'anhydride acétique utilisé comme solvant, en aminomalonitrile quen l'on précipite sous forme d'acétylaminomalonitrile et isole.

2. Procédé selon la revendication 1, caractérisé en ce qu'on utilise des catalyseurs de Raney contenant Ni, Fe, Cu et/ou Co.

3. Procédé selon la revendication 1 ou 2, caractérisé en ce que l'on utilise 60 á 80, de préférence 70 g de catalyseur par mole de malonitrile.

4. Procédé selon l'une quelconque des revendications 1 á 3,caractérisé en ce que l'on ajoute l'acide p-toluéne sulfonique au mélange réactionnel en deux fractions, á savoir une premiére fraction de 0,02 mole á 0,03 mole d'acide p-toluénesulfonique et une deuxiéme fraction de 0,03 mole á 0,1 mole d'acide p-toluénesulfonique par rapport á 0,1 mole de malonitrile.

**Claims**

1. Method of producing aminomalonic acid dinitrile tosylate or acetylaminomalonic acid dinitrile from a malonic acid dinitrile compound of the general formula

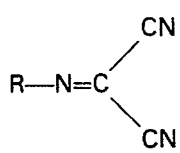

wherein R signifies —OH or

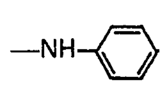

by reduction, characterised in that the operation is carried out in the presence of Raney catalysts as the reducing agents at hydrogen pressures of ~ 5—7 bars (4 to 6 atmospheres) and temperatures from 10°C to 80°C in tetrahydrofuran as the solvent, and the aminomalonic acid dinitrile is precipitated from the reaction solution by being treated with p-toluene sulphonic acid and is isolated as tosylate or reacts

in acetanhydride as the solvent to form aminomalonic acid dinitrile and this is precipitated and isolated as acetylaminomalonic acid dinitrile.

2. Method as claimed in claim 1, characterised in that Raney catalysts are used which contain Ni, Fe, Cu and/or Co.

3. Method as claimed in claims 1 and 2, characterised in that 60 g to 80 g, preferably 70 g, of catalyst is used per mole of malonic acid dinitrile compound.

4. Method as claimed in claims 1 to 3, characterised in that p-toluene sulphonic acid is added in two fractions to the reaction mixture, 0.02 mole to 0.03 mole of p-toluene sulphonic acid being used in the first fraction and 0.03 mole to 0.1 mole of p-toluene sulphonic acid being used in the second fraction, based on 0.1 mole of malonic acid dinitrile compound.